# EUROPEAN PATENT SPECIFICATION

(11) **EP 1 770 083 B1**
(45) Date of publication and mention of the grant of the patent: **29.04.2009**
(21) Application number: 07000764.6
(22) Date of filing: 30.04.1997
(51) Int. Cl.: C07C 211/27, C07C 211/28, C07C 211/30, A61K 31/135

(54) **Inorganic ion receptor-active compounds**
Inorganische am Ionen-Rezeptor aktive Verbindungen
Composés servant a déclencher l'activité d'un recepteur d'ions inorganiques

(30) Priority: 01.05.1996 US 16673 P
(43) Date of publication of application: 04.04.2007
(62) Divisional of application: 02018228.3
(73) Proprietor: NPS PHARMACEUTICALS, INC., Salt Lake City, UT 84108 (US)
(72) Inventor: Moe, Scott T., Marlboro MA 01752 (US); Van Waganen, Bradford C., Salt Lake City UT 84124 (US); Delmar, Eric G., Salt Lake City UT 84108 (US); Trovato, Richard, Salt Lake City UT 84117 (US); Balandrin, Manuel F., Sandy UT 84093 (US)
(74) Representative: Bradley, Adrian

(56) References cited:
- EP-A- 0 092 787
- WO-A-93/04373
- WO-A-94/18959
- WO-A-95/11221
- WO-A-95/18134
- WO-A-96/02492
- WO-A-96/12697
- DE-A- 3 541 181
- ANTON STÜTZ ET AL.: "Synthesis and Structure-Activity Relationships of Naftifine-Related Allylamine Antimyotics" JOURNAL OF MEDICINAL CHEMISTRY, vol. 29, no. 1, January 1986 (1986-01), pages 112-125, XP002037080 WASHINGTON US
- DATABASE CROSSFIRE Beilstein Informationssysteme GmbH, Frankfurt DE; XP002037081 & J. CHEM. RES. MINIPRINT, vol. 10, 1981, pages 3529-3549,
- DATABASE CROSSFIRE Beilstein Informationssysteme GmbH, Frankfurt DE; XP002037082 & ARZNEIM. FORSCH., vol. 17, 1967, pages 1145-1149,
- DATABASE CROSSFIRE Beilstein Informationssysteme GmbH, Frankfurt DE; XP002037083 & J. CHEM. SOC. CHEM. COMMUN., 1995, pages 1421-1422,
- DATABASE CROSSFIRE Beilstein Informationssysteme GmbH, Frankfurt DE; XP002037084 & CHEM. PHARM. BULL., vol. 31, 1983, pages 3471-3485,
- DATABASE CROSSFIRE Beilstein Informationssysteme GmbH, Frankfurt DE; XP002037085 & ARCH. PHARM., vol. 326, 1993, pages 341-350,
- YUKIHIKO HASHIMOTO ET AL: "Highly Diastereoselective Addition of Organometallic Reagents to Chiral Imines Derived from 1-(2-Methoxyphenyl)ethylamine" SYNLETT, THIEME INTERNATIONAL, STUTTGART, DE, no. 9, September 1995 (1995-09), pages 961-962, XP002217353 ISSN: 0936-5214

## Description

### FIELD OF THE INVENTION

This invention relates to compounds able to modulate one or more inorganic ion receptor activities.

### BACKGROUND OF THE INVENTION

Certain cells in the body respond not only to chemical signals, but also to ions such as extracellular calcium ions (Ca²⁺). Extracellular Ca²⁺ is under tight homeostatic control and regulates various processes such as blood clotting, nerve and muscle excitability, and proper bone formation.

Calcium receptor proteins enable certain specialized cells to respond to changes in extracellular Ca²⁺ concentration. For example, extracellular Ca²⁺ inhibits the secretion of parathyroid hormone (PTH) from parathyroid cells, inhibits bone resorption by osteoclasts, and stimulates secretion of calcitonin from C-cells.

PTH is the principal endocrine factor regulating Ca²⁺ homeostasis in the blood and extracellular fluids. PTH, by acting on bone and kidney cells, increases the level of Ca²⁺ in the blood. This increase in extracellular Ca²⁺ then acts as a negative feedback signal, depressing PTH secretion. The reciprocal relationship between extracellular Ca²⁺ and PTH secretion forms an important mechanism maintaining bodily Ca²⁺ homeostasis.

Extracellular Ca²⁺ acts directly on parathyroid cells to regulate PTH secretion. The existence of a parathyroid cell surface protein which detects changes in extracellular Ca²⁺ has been confirmed. (Brown et al., Nature 366:574, 1993.) In parathyroid cells, this protein, the calcium receptor, acts as a receptor for extracellular Ca²⁺, detects changes in the ion concentration of extracellular Ca²⁺, and initiates a functional cellular response, PTH secretion.

Extracellular Ca²⁺ can exert effects on different cell functions, reviewed in Nemeth et al., Cell Calcium 11:319, 1990. The role of extracellular Ca²⁺ in parafollicular (C-cells) and parathyroid cells is discussed in Nemeth, Cell Calcium 11:323, 1990. These cells were shown to express similar calcium receptors. (See Brown et al., Nature 366:574, 1993; Mithal et al., J. Bone Miner. Res. 9, Suppl. 1, s2B2, 1994; Rogers et al., J. Bone Miner. Res. 9, Suppl, 1, s409, 1994; Garrett et al., Endocrinology 136:5202-5211, 1995.)

The ability of various molecules to mimic extracellular Ca²⁺ *in vitro* is discussed in references such as Nemeth et al., in "Calcium-Binding Proteins in Health and Disease," 1987,. Academic Press, Inc., pp. 33-35; Brown et al., Endocrinology 128:3047, 1991; Chen et al., J. Bone Miner. Res. 5:581, 1990; and Zaidi et al., Biochem. Biophys. Res. Commun. 167:807, 1990.

Nemeth et al., PCT/US92/07175, International Publication Number WO 93/04373, Nemeth et al., PCT/US93/01642, International Publication Number WO 94/18959, and Nemeth et al., PCT/US94/12117, International Publication Number WO 95/11211, describe various compounds which can modulate the effect of an inorganic ion receptor.

### SUMMARY OF THE INVENTION

The present invention features compounds able to modulate one or more activities of an inorganic ion receptor and such compounds for use in methods for treating diseases or disorders using such compounds. Preferred compounds can mimic or block the effect of extracellular calcium on a cell surface calcium receptor.

Inorganic ion receptor activities are those processes brought about as a result of inorganic ion receptor activation. Such processes include the production of molecules which can act as intracellular or extracellular messengers.

Inorganic ion receptor-modulating compounds include ionomimetics, ionolytics, calcimimetics, and calcilytics. Ionomimetics are compounds which mimic (*i.e.*, evoke or potentiate) the effects of an inorganic ion at an inorganic ion receptor. Preferably, the compound affects one or more calcium receptor activities. Calcimimetics are ionomimetics which affect one or more calcium receptor activities.

Ionolytics are compounds which block (*i.e.*, inhibit or diminish) one or more activities caused by an inorganic ion at an inorganic ion receptor. Preferably, the compound affects one or more calcium receptor activities. Calcilytics are ionolytics which block one or more calcium receptor activities evoked by extracellular calcium.

Ionomimetics and ionolytics may bind at the same receptor site as the native inorganic ion ligand binds or can bind at a different site (*e.g.*, an allosteric site). For example, NPS R-467 binding to a calcium receptor results in calcium receptor activity and, thus, NPS R-467 is classified as a calcimimetic. However, NPS R-467 binds to the calcium receptor at a different site (*i.e.*, an allosteric site) than extracellular calcium.

A measure of the effectiveness of a compound to modulate receptor activity can be determined by calculating the EC₅₀ or IC₅₀ for that compound. The EC₅₀ is the concentration of a compound which causes a half-maximal mimicking effect. The IC₅₀ is the concentration of a compound which causes a half-maximal blocking effect. EC₅₀ and IC₅₀ values for compounds at a calcium receptor can be determined by assaying one or more of the activities of extracellular calcium at a calcium receptor. Examples of assays for measuring EC₅₀ and IC₅₀ values are described Nemeth et al., PCT/US93/01642, International Publication Number WO 94/18959, and Nemeth et al., PCT/US92/07175, International Publication Number WO 93/04373. Such assays include oocyte expression assays and measuring increases in intracellular calcium ion concentration ([Ca²⁺]ᵢ) due to calcium receptor activity. Preferably, such assays measure the release or inhibition of a particular hormone associated with activity of a calcium receptor.

An inorganic ion receptor-modulating compound preferably selectively targets inorganic ion receptor activity in a particular cell. For example, selective targeting of a calcium receptor activity is achieved by a compound exerting a greater effect on a calcium receptor activity in one cell type than at another cell type for a given concentration of compound. Preferably, the differential effect is 10-fold or greater as measured *in vivo* or *in vitro.* More preferably, the differential effect is measured *in vivo* and the compound concentration is measured as the plasma concentration or extracellular fluid concentration and the measured effect is the production of extracellular messengers such as plasma calcitonin, parathyroid hormone, or plasma calcium. For example, in a preferred embodiment, the compound selectively targets PTH secretion over calcitonin secretion.

Preferably, the compound is either a calcimimetic or calcilytic having an EC₅₀ or an IC₅₀ at a calcium receptor of less than or equal to 5 µM, and even more preferably less than or equal to 1 µM, 100 nmolar, 10 nmolar, or 1 nmolar using one of the assays described below. More preferably, the assay measures intracellular Ca²⁺ in HEK 293 cells transformed with nucleic acid expressing the human parathyroid calcium receptor and loaded with fura-2. Lower EC₅₀ or IC₅₀ values are advantageous since they allow lower concentrations of compounds to be used *in vivo* or *in vitro*. The discovery of compounds with low EC₅₀ and IC₅₀ values enables the design and synthesis of additional compounds having similar or improved potency, effectiveness, and/or selectivity.

Thus, a first aspect the invention features an inorganic ion receptor-modulating compound having the formula: 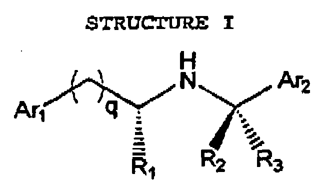 wherein Ar₁ is either optionally substituted naphthyl, or an optionally substituted heterocyclic aryl, where up to 5 substituents may be present and each substituent is independently selected from the group consisting of: alkyl, alkenyl, halogen, alkoxy, thioalkyl, methylene dioxy, haloalkyl, haloalkoxy, OH, CH₂OH, CONH₂, CN, acetoxy, N(alkyl)₂, phenyl, phenoxy, benzyl, benzyloxy, α,α-dimethylbenzyl, NO₂, CHO, CH₃CH(OH), acetyl, OCH₂COOH, and ethylene dioxy;
Ar₂ is either optionally substituted naphthyl, or an optionally substituted heterocyclic aryl, where up to 5 substituents may be present and each substituent is independently selected from the group consisting of: alkyl, alkenyl, halogen, alkoxy, thioalkyl, methylene dioxy, haloalkyl, haloalkoxy, OH, CH₂OH, CONH₂, CN, OCH₂COOH, ethylene dioxy, and acetoxy;
wherein one or both of Ar, and Ar₂ are heterocyclic aryl; q is 0, 1, 2, or 3;
R₁ is either H or alkyl; and
R₂ and R₃ are each independently either hydrogen, alkyl, or together cycloalkyl or cycloalkenyl;
and pharmaceutically acceptable salts and complexes thereof.

Wherein, the compound is an ionomimetic which modulates one or more inorganic ion receptor activities, more preferably the compound is a calcimimetic.

"Alkenyl" refers to a hydrocarbon chain having 2-6 carbons and at least one double-bond which may be a straight chain, branched, or non-aromatic cyclic. Preferably, the alkenyl has 2-4 carbon atoms.

"Alkyl" refers to a saturated hydrocarbon having 1-6 carbons which may be a straight chain, branched, or cyclic. Preferably, the alkyl has 1-4 carbon atoms.

"Alkoxy" refers to "O-alkyl," where "O" is an oxygen joined to an alkyl.

"Cycloalkenyl" refers to a non-aromatic cyclic hydrocarbon chain having 3-12 carbons and at least one double-bond, and includes multiple ring structures. Preferably, the cycloalkenyl has 3 to 6 carbon atoms.

"Cycloalkyl" refers to a saturated cyclic hydrocarbon chain having 3-12 carbons, and includes multiple ring structures. Preferably, the cycloalkyl has 3 to 6 carbon atoms.

"Thioalkyl" refers to "S-alkyl," where "S" is a sulfur joined to an alkyl.

"Haloalkyl" refers to an alkyl substituted with at least one halogen. Preferably, only the terminal carbon of the haloalkyl is substituted with a halogen and 1 to 3 halogens are present. More preferably, the haloalkyl contains 1 carbon. Preferably, the halogen substitutions are either Cl or F.

"Haloalkoxy" refers to "O-haloalkyl," where "O" is an oxygen joined to a haloalkyl.

"Heterocyclic aryl" refers to an aryl ring system having 1 to 3 heteroatoms as ring atoms in a heteroaromatic ring system and the remainder of the ring atoms are carbon atoms. Suitable heteroatoms include oxygen, sulfur, and nitrogen. Preferably, the heterocyclic ring system is mono- or bicyclic. More preferably, the heterocyclic aryl is either furanyl, thiofuranyl (also known as "thienyl"), benzofuranyl or benzothiofuranyl (also known as "benzothienyl").

Another aspect of the present invention features a pharmaceutical composition made up of an inorganic ion receptor-modulating compound described herein and a physiologically acceptable carrier. A "pharmacological composition" refers to a composition in a form suitable for administration into a mammal, preferably a human.
Preferably, the pharmaceutical composition contains a sufficient amount of a calcium receptor-modulating compound in a proper pharmaceutical form to exert a therapeutic effect on a human.

Considerations concerning forms suitable for administration are known in the art and include toxic effects, solubility, route of administration, and maintaining activity. For example, pharmacological compositions injected into the blood stream should be soluble.

Pharmaceutical compositions can also be formulated as pharmaceutically acceptable salts (*e.g.,* acid addition salts) and complexes thereof. The preparation of such salts can facilitate the pharmacological use of a compound by altering its physical characteristics without preventing it from exerting a physiological effect.

Also described is a method involving administering to the patient a pharmaceutical composition containing a therapeutically effective amount of an inorganic ion receptor-modulating compound. In a preferred embodiment, the disease or disorder is treated by administering to the patient a therapeutically effective amount of a calcium receptor-modulating compound.

Inorganic ion receptor-modulating compounds, and compositions containing such compounds, can be used to treat different types of patients. A "patient" refers to a mammal in which compounds able to modulate inorganic ion receptor activity will have a beneficial effect including a beneficial prophylactic effect. Suitable patients can be diagnosed using standard techniques known to those in the medical profession.

Preferably, a patient is a human having a disease or disorder characterized by one more of the following: (1) abnormal inorganic ion homeostasis, more preferably abnormal calcium homeostasis; (2) an abnormal level of a messenger whose production or secretion is affected by inorganic ion receptor activity, more preferably affected by calcium receptor activity; and (3) an abnormal level or activity of a messenger whose function is affected by inorganic ion receptor activity, more preferably affected by calcium receptor activity.

Diseases characterized by abnormal calcium homeostasis include hyperparathyroidism, osteoporosis and other bone and mineral-related disorders, and the like (as described, *e.g.*, in standard medical text books, such as "Harrison's Principles of Internal Medicine"). Such diseases are treated using calcium receptor-modulating compounds which mimic or block one or more of the effects of extracellular Ca²⁺ on a calcium receptor.

By "therapeutically effective amount" is meant an amount of a compound which relieves to some extent one or more symptoms of a disease or disorder in the patient; or returns to normal either partially or completely one or more physiological or biochemical parameters associated with or causative of the disease or disorder. Thus, a therapeutically effective amount can be an amount effective to prophylactically decrease the likelihood of the onset of a disease or disorder.

In a preferred embodiment, the patient has a disease or disorder characterized by an abnormal level of one or more calcium receptor-regulated components and the compound is active on a calcium receptor of a cell selected from the group consisting of: parathyroid cell, bone osteoclast, juxtaglomerular kidney cell, proximal tubule kidney cell, distal tubule kidney cell, central nervous system cell, peripheral nervous system cell, cell of the thick ascending limb of Henle's loop and/or collecting duct, keratinocyte in the epidermis, parafollicular cell in the thyroid (C-cell), intestinal cell, platelet, vascular smooth muscle cell, cardiac atrial cell, gastrin-secreting cell, glucagon-secreting cell, kidney mesangial cell, mammary cell, beta cell, fat/adipose cell, immune cell, GI tract cell, skin cell, adrenal cell, pituitary cell, hypothalamic cell, and cell of the subfornical organ.

More preferably, the cells are chosen from the group consisting of: parathyroid cell, central nervous system cell, peripheral nervous system cell, cell of the thick ascending limb of Henle's loop and/or collecting duct in the kidney, parafollicular cell in the thyroid (C-cell), intestinal cell, GI tract cell, pituitary cell, hypothalamic cell, and cell of the subfornical organ.

In a preferred embodiment, the compound reduces the level of parathyroid hormone in the serum of the patient. More preferably, the level is reduced to a degree sufficient to cause a decrease in plasma Ca²⁺. Most preferably, the parathyroid hormone level is reduced to that present in a normal individual.

Patients in need of treatment using the compounds described by the present invention can be diagnosed by standard medical techniques, such as blood or urine analysis. Examples of such medical techniques include detecting a deficiency of protein whose production or secretion is affected by changes in inorganic ion concentrations, and by detecting abnormal levels of inorganic ions or hormones which effect inorganic ion homeostasis.

### BRIEF DESCRIPTION OF THE DRAWING

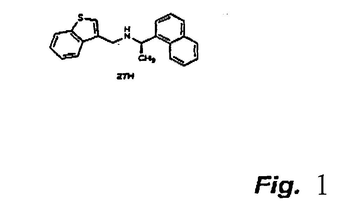

Figure 1 provides the chemical structures of different ionomimetic compounds.

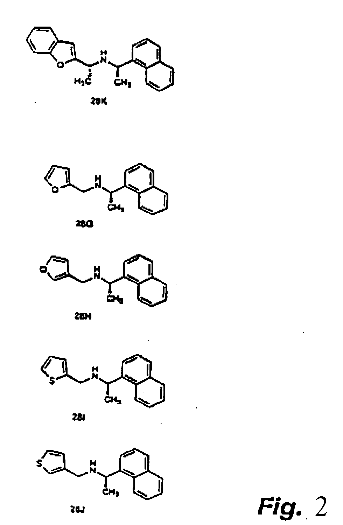

Figure 2 provides the chemical structures of different ionomimetic compounds.

### DESCRIPTION OF THE PREFERRED EMBODIMENTS

The present invention features compounds able to modulate one or more inorganic ion receptor activities. Preferably, the compounds can mimic or block an effect of an extracellular ion on a cell having an inorganic ion receptor, more preferably, the extracellular ion is Ca²⁺ and the effect is on a cell having a calcium receptor. Most preferably, the compounds can mimic the effect of extracellular Ca²⁺ on a cell having a calcium receptor.

### I. CALCIUM RECEPTORS

Calcium receptors are present in different cells. The pharmacological effects of the following cells, in response to extracellular Ca²⁺, is consistent with the presence of a calcium receptor: parathyroid cell, bone osteoclast, juxtaglomerular kidney cell, proximal tubule kidney cell, distal tubule kidney cell, central nervous system cell, peripheral nervous system cell, cell of the thick ascending limb of Henle's loop and/or collecting duct, keratinocyte in the epidermis, parafollicular cell in the thyroid (C-cell), intestinal cell, trophoblast in the placenta, platelet, vascular smooth muscle cell, cardiac atrial cell, gastrin-secreting cell, glucagon-secreting cell, kidney mesangial cell, mammary cell, endocrine and exocrine cells in the pancreas, fat/adipose cell, immune cell, GI tract cell, skin cell, adrenal cell, pituitary cell, hypothalamic cell, and cell of the subfornical organ.

The presence of a calcium receptor on the following cells have been confirmed using physical data, such as hybridization with nucleic acid encoding a calcium receptor: parathyroid cell, central nervous system cell, peripheral nervous system cell, cell of the thick ascending limb of Henle's loop and/or collecting duct in the kidney, parafollicular cell in the thyroid (C-cell), intestinal cell, GI tract cell, pituitary cell, hypothalamic cell, cell of the subfornical organ, and endocrine and exocrine cells in the pancreas.

The calcium receptor on these different cell types may be different. It is also possible that a cell can have more than one type of calcium receptor. Comparison of calcium receptor activities and amino acid sequences from different cells indicate that distinct calcium receptor types exist. For example, calcium receptors can respond to a variety of di- and trivalent cations. The parathyroid cell calcium receptor responds to calcium and Gd³⁺, while osteoclasts respond to divalent cations such as calcium, but do not respond to Gd³⁺. Thus, the parathyroid cell calcium receptor is pharmacologically distinct from the calcium receptor on the osteoclast.

On the other hand, the nucleic acid sequences encoding calcium receptors present in parathyroid cells and C-cells indicate that these receptors have a very similar amino acid structure. Nevertheless, calcimimetic compounds exhibit differential pharmacology and regulate different activities at parathyroid cells and C-cells. Thus, pharmacological properties of calcium receptors may vary significantly depending upon the cell type or organ in which they are expressed even though the calcium receptors may have similar or even identical structures.

Calcium receptors, in general, have a low affinity for extracellular Ca²⁺ (apparent K_{d} generally greater than about 0.5 mM). Calcium receptors may include a free or bound effector mechanism as defined by Cooper, Bloom and Roth, "The Biochemical Basis of Neuropharmacology", Ch. 4, and are thus distinct from intracellular calcium receptors, *e.g.*, calmodulin and the troponins.

Calcium receptors respond to changes in extracellular calcium levels. The exact changes depend on the particular receptor and cell line containing the receptor. For example, the *in vitro* effect of calcium on the calcium receptor in a parathyroid cell includes the following:
1. An increase in internal calcium. The increase is due to the influx of external calcium and/or to mobilization of internal calcium. Characteristics of the increase in internal calcium include the following:
   (a) A rapid (time to peak < 5 seconds) and transient increase in [Ca²⁺]ᵢ that is refractory to inhibition by 1 µM La³⁺ or 1 µM Gd³⁺ and is abolished by pretreatment with ionomycin (in the absence of extracellular Ca²⁺);
   (b) The increase is not inhibited by dihydropyridines;
   (c) The transient increase is abolished by pretreatment for 10 minutes with 10 mM sodium fluoride;
   (d) The transient increase is diminished by pretreatment with an activator of protein kinase C (PKC), such as phorbol myristate acetate (PMA), mezerein or (-)-indola tam V. The overall effect of the protein kinase C activator is to shift the concentration-response curve of calcium to the right without affecting the maximal response; and
   (e) Pretreatment with pertussis toxin (100 ng/ml for > 4 hours) does not affect the increase.
2. A rapid (< 30 seconds) increase in the formation of inositol-1,4,5-triphosphate or diacylglycerol. Pretreatment with pertussis toxin (100 ng/ml for > 4 hours) does not affect this increase;
3. The inhibition of dopamine- and isoproterenol-stimulated cyclic AMP formation. This effect is blocked by pretreatment with pertussis toxin (100 ng/ml for > 4 hours); and
4. The inhibition of PTH secretion. Pretreatment with pertussis toxin (100 ng/ml for > 4 hours) does not affect the inhibition in PTH secretion.

Using techniques known in the art, the effect of calcium on other calcium receptors in different cells can be readily determined. Such effects may be similar in regard to the increase in internal calcium observed in parathyroid cells. However, the effect is expected to differ in other aspects, such as causing or inhibiting the release of a hormone other than parathyroid hormone.

### II. INORGANIC ION RECEPTOR-MODULATING COMPOUNDS

Inorganic ion receptor-modulating compounds modulate one or more inorganic ion receptor activities. Preferred inorganic ion receptor-modulating compounds are calcimimetics or calcilytics. Inorganic ion receptor-modulating compounds can be identified by screening compounds which are modeled after a compound shown to have a particular activity (*i.e.*, a lead compound).

A preferred method of measuring calcium receptor activity is to measure changes in [Ca²⁺]ᵢ. Changes in [Ca²⁺]ᵢ can be measured using different techniques such as by using HEK 293 cells transduced with nucleic acid expressing the human parathyroid calcium receptor and loaded with fura-2; and by measuring an increase in Cl⁻ current in a *Xenopus* oocyte injected with nucleic acid coding for a calcium receptor. (*See* Nemeth *et al.*, PCT/US93/01642, International Publication Number WO 94/18959.) For example, poly(A)⁺ mRNA can be obtained from cells expressing a calcium receptor, such as a parathyroid cell, bone osteoclast, juxtaglomerular kidney cell, proximal tubule kidney cell, distal tubule kidney cell, cell of the thick ascending limb of Henle's loop and/or collecting duct, keratinocyte in the epidermis, parafollicular cell in the thyroid (C-cell), intestinal cell, central nervous cell, peripheral nervous system cell, platelet, vascular smooth muscle cell, cardiac atrial cell, gastrin-secreting cell, glucagon-secreting cell, kidney mesangial cell, mammary cell, beta cell, fat/adipose cell, immune cell, and GI tract cell. Preferably, the nucleic acid is from a parathyroid cell, C-cell, or osteoclast. More preferably, the nucleic acid encodes a calcium receptor and is present on a plasmid or vector.

In a preferred embodiment, the compound has an EC₅₀ or IC₅₀ less than or equal to 5 µM at one or more, but not all cells chosen from the group consisting of: parathyroid cell, bone osteoclast, juxtaglomerular kidney cell, proximal tubule kidney cell, distal tubule kidney cell, central nervous system cell, peripheral nervous system cell, cell of the thick ascending limb of Henle's loop and/or collecting duct, keratinocyte in the epidermis, parafollicular cell in the thyroid (C-cell), intestinal cell, platelet, vascular smooth muscle cell, cardiac atrial cell, gastrin-secreting cell, glucagon-secreting cell, kidney mesangial cell, mammary cell, beta cell, fat/adipose cell, immune cell, GI tract cell, skin cell, adrenal cell, pituitary cell, hypothalamic cell, and cell of the subfornical organ. More preferably, the cells are chosen from the group consisting of parathyroid cell, central nervous system cell, peripheral nervous system cell, cell of the thick ascending limb of Henle's loop and/or collecting duct in the kidney, parafollicular cell in the thyroid (C-cell), intestinal cell, GI tract cell, pituitary cell, hypothalamic cell, and cell of the subfornical organ. The presence of a calcium receptor in this group of cells has been confirmed by physical data such as in situ hybridization and antibody staining.

Preferably, inorganic ion receptor-modulating compounds mimic or block the effects of an extracellular ion on a cell having an inorganic ion receptor, such that the compounds achieve a therapeutic effect. Inorganic ion receptor-modulating compounds may have the same, or different, effects on cells having different types of inorganic ion receptor morphology (*e.g.*, such as cells having normal inorganic ion receptors, a normal number of inorganic ion receptors, an abnormal inorganic ion receptor, and an abnormal number of inorganic ion receptors).

Calcium receptor-modulating compounds preferably mimic or block all of the effects of extracellular ion in a cell having a calcium receptor. However, calcimimetics need not possess all the biological activities of extracellular Ca²⁺. Similarly, calcilytics need not block all of the activities caused by extracellular calcium. Additionally, different calcimimetics and different calcilytics do not need to bind to the same site on the calcium receptor as does extracellular Ca²⁺ to exert their effects.

Inorganic receptor-modulating compounds need not effect inorganic receptor activity to the same extent or in exactly the same manner as the natural ligand. For example, a calcimimetic may affect calcium receptor activity to a different extent, to a different duration, by binding to a different binding site, or by having a different affinity, compared to calcium acting at a calcium receptor.

### A. Ionomimetics

Different compound are described by Nemeth et al., PCT/US92/07175, International Publication Number WO 93/04373, Nemeth et al., PCT/US93/01642, International Publication Number WO 94/18959, Nemeth et al., PCT/US94/12117, International Publication Number WO 95/11211, and Van Wagenen et al. PCT/US95/13704.

### 1. Structure I Compounds

Structure I compounds able to modulate calcium receptor activity have the following formula:

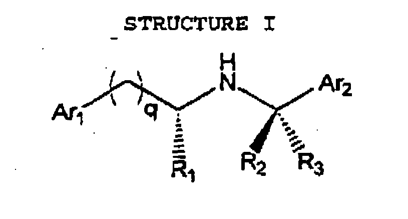

Where Ar₁ is either optionally substituted naphthyl, or an optionally substituted heterocyclic aryl, where up to 5 substituents may be present and each substituent is independently selected from the group consisting of: alkyl, alkenyl, halogen, alkoxy, thioalkyl, methylene dioxy, haloalkyl, haloalkoxy, OH, CH₂OH, CONH₂, CN, acetoxy, N(alkyl)₂, phenyl, phenoxy, benzyl, benzyloxy, α,α-dimethylbenzyl, NO₂, CHO, CH₃CH(OH), acetyl, OCH₂COOH, and ethylene dioxy. In one embodiment of the present invention Ar₁ is either an optionally substituted naphthyl, having 1 to 4 substituents, more preferably Ar₁ is an unsubstituted naphthyl preferably each Ar₁ substituent is independently selected from the group consisting of: isopropyl, CH₃O, CF₃ CH₃S, CF₃O, Br, I, Cl, F, and CH₃. In another embodiment of the present invention Ar₁ is an optionally substituted heterocyclic aryl. Preferred heterocyclic aryl substituents are independently selected from the group consisting of: isopropyl, CH₃O, CF₃ CH₃S, CF₃O, Br, I, Cl, F, and CH₃. Preferred heterocyclic aryls are either furanyl, thiofuranyl, benzofuranyl, or benzothiophenyl;
Ar₂ is either optionally substituted naphthyl, or an optionally substituted heterocyclic aryl, where up to 5 substituents may be present and each substituent is independently selected from the group consisting of: alkyl, alkenyl, halogen, alkoxy, thioalkyl, methylene dioxy, haloalkyl, haloalkoxy, OH, CH₂OH, CONH₂, CN, OCH₂COOH, ethylene dioxy, and acetoxy; In one embodiment Ar₂ is preferably an optionally substituted naphthyl, more preferably Ar₂ is an unsubstituted naphthyl; preferably each Ar₂ substituent is independently selected from the group consisting of: isopropyl, CH₃O, CH₃S, CF₃O, Br, I, Cl, F, CF₃, and CH₃, more preferably a CH₃O is located in the meta position. In another embodiment of the present invention Ar₂ is an optionally substituted heterocyclic aryl. Preferred heterocyclic aryl substituents are independently selected from the group consisting of: isopropyl, CH₃O, CF₃ CH₃S, CF₃O, Br, I, Cl, F, and CH₃. Preferred heterocyclic aryls are either furanyl, thiofuranyl, benzofuranyl, or benzothiophenyl;
wherein one or both of Ar, and Ar₂ are heterocyclic aryl;
q is 0, 1, 2, or 3; in alternative embodiments q is 0 or 2;
R₁ is either H or alkyl; when R₁ is alkyl in alternative embodiments the alkyl is methyl, or the alkyl has more than one carbon atom, preferably 2 to 4 carbon atoms;
R₂ and R₃ are each independently either hydrogen, alkyl, or together cycloalkyl or cycloalkenyl; preferably, R₂ and R₃ are each independently either hydrogen or alkyl, provided that at least one of R₂ and R₃ is not hydrogen, preferably, R₂ is alkyl, more preferably R₂ is methyl;
and pharmaceutically acceptable salts and complexes thereof.

In a more preferred embodiment the compound has following formula:

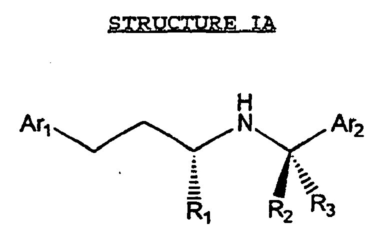

Where Ar₁, Ar₂, R₁, R₂, and R₃ are as described above for Structure I compounds, including preferred embodiments.

In another more preferred embodiment the compound has the formula:

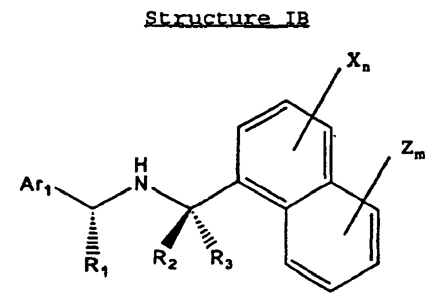

Where Ar₁, R₁, R₂, and R₃ is as described above for Structure I compounds including preferred embodiments;
each X and Z is independently selected from the group consisting of: alkyl, alkenyl, halogen, alkoxy, thioalkyl, methylene dioxy, haloalkyl, haloalkoxy, OH, CH₂OH, CONH₂, CN, OCH₂COOH, ethylene dioxy, and acetoxy; more preferably each X and Z is independently selected from the group consisting of: isopropyl, CH₃O, CH₃S, CF₃O, Br, I, Cl, F, CF₃, and CH₃;
n and m are each independently 0, 1, 2, or 3, provided that n and m together are no more than 5; preferably n and m are each independently 0 or 1, more preferably, 0.

### 3. Calcimimetic Activity

The ability of compounds to mimic the activity of Ca²⁺ at calcium receptors can be determined using procedures known in the art such as those described by Nemeth *et al.*, PCT/US93/01642, International Publication Number WO 94/18959. For example, calcimimetics possess one or more and preferably all of the following activities when tested on parathyroid cells *in vitro:*
1. The compound causes a rapid (time to peak < 5 seconds) and transient increase in intracellular calcium concentration that is refractory to inhibition by 1 µM La³⁺ or 1 µM Gd³⁺. The increase in [Ca²⁺]ᵢ persists in the absence of extracellular Ca²⁺, but is abolished by pretreatment with ionomycin (in the absence of extracellular Ca²⁺);
2. The compound potentiates increases in [Ca²⁺]ᵢ elicited by submaximal concentrations of extracellular Ca²⁺;
3. The increase in [Ca²⁺]ᵢ elicited by extracellular Ca²⁺ is not inhibited by dihydropyridines;
4. The transient increase in [Ca²⁺]ᵢ caused by the compound is abolished by pretreatment for 10 minutes with 10 mM sodium fluoride;
5. The transient increase in [Ca²⁺]ᵢ caused by the compound is diminished by pretreatment with an activator of protein kinase C (PKC), such as phorbol myristate acetate (PMA), mezerein or (-)-indolactam V. The overall effect of the protein kinase C activator is to shift the concentration-response curve of the compound to the right without affecting the maximal response;
6. The compound causes a rapid (< 30 seconds) increase in the formation of inositol-1,4,5-triphosphate and/or diacylglycerol;
7. The compound inhibits dopamine- or isoproterenol-stimulated cyclic AMP formation;
8. The compound inhibits PTH secretion;
9. Pretreatment with pertussis toxin (100 ng/ml for > 4 hours) blocks the inhibitory effect of the compound on cyclic AMP formation, but does not effect increases in [Ca²⁺]ᵢ, inositol-1,4,5-triphosphate, or diacylglycerol, nor decreases in PTH secretion;
10. The compound elicits increases in Cl- current in *Xenopus* oocytes injected with poly (A)⁺-enriched mRNA from bovine or human parathyroid cells, but is without effect in *Xenopus* oocytes injected with water, or liver mRNA; and
11. Similarly, using a cloned calcium receptor from a parathyroid cell, the compound will elicit a response in *Xenopus* oocytes injected with the specific cDNA or mRNA encoding the receptor.

Different calcium activities can be measured using available techniques. Parallel definitions of compounds mimicking Ca²⁺ activity on other calcium responsive cell, preferably at a calcium receptor, are evident from the examples provided herein and Nemeth et al., PCT/US93/01642, International Publication Number WO 94/18959.

Preferably, the compound as measured by the bioassays described herein, or by Nemeth et al., PCT/US93/01642, International Publication Number WO 94/18959, has one or more, more preferably all of the following activities: evokes a transient increase in internal calcium, having a duration of less that 30 seconds (preferably by mobilizing internal calcium); evokes a rapid increase in [Ca²⁺]ᵢ, occurring within thirty seconds; evokes a sustained increase (greater than thirty seconds) in [Ca²⁺]ᵢ (preferably by causing an influx of external calcium); evokes an increase in inositol-1,4,5-triphosphate or diacylglycerol levels, preferably within less than 60 seconds; and inhibits dopamine- or isoproterenol-stimulated cyclic AMP formation.

The transient increase in [Ca²⁺]ᵢ is preferably abolished by pretreatment of the cell for ten minutes with 10 mM sodium fluoride, or the transient increase is diminished by brief pretreatment (not more than ten minutes) of the cell with an activator of protein kinase C, preferably, phorbol myristate acetate (PMA), mezerein or (-) indolactam v.

### B. Calcilytics

The ability of a compound to block the activity of extracellular calcium at a calcium receptor can be determined using standard techniques based on the present disclosure. (*See,* also Nemeth et al., PCT/US93/01642, International Publication Number WO 94/18959.) For example, compounds which block the effect of extracellular calcium, when used in reference to a parathyroid cell, possess one or more, and preferably all of the following characteristics when tested on parathyroid cells *in vitro:*
1. The compound blocks, either partially or completely, the ability of increased concentrations of extracellular Ca²⁺ to:
   (a) increase [Ca²⁺]ᵢ,
   (b) mobilize intracellular Ca²⁺,
   (c) increase the formation of inositol-1,4,5-triphosphate,
   (d) decrease dopamine- or isoproterenol-stimulated cyclic AMP formation, and
   (e) inhibit PTH secretion;
2. The compound blocks increases in Cl⁻ current in *Xenopus* oocytes injected with poly(A)⁺-mRNA from bovine or human parathyroid cells elicited by extracellular Ca²⁺ or calcimimetic compounds, but not in *Xenopus* oocytes injected with water or liver mRNA;
3. Similarly, using a cloned calcium receptor from a parathyroid cell, the compound will block a response in *Xenopus* oocytes injected with the specific cDNA, mRNA or cRNA encoding the calcium receptor, elicited by extracellular Ca²⁺ or a calcimimetic compound.

Parallel definitions of compounds blocking Ca²⁺ activity on a calcium responsive cell, preferably at a calcium receptor, are evident from the examples provided herein and Nemeth et al., PCT/US93/01642, International Publication Number WO 94/18959.

### III. TREATMENT OF DISEASES OR DISORDERS

Diseases or disorders which can be treated using compounds able to modulate inorganic ion receptor activity include one or more of the following types: (1) those characterized by abnormal inorganic ion homeostasis, preferably calcium homeostasis; (2) those characterized by an abnormal amount of an extracellular or intracellular messenger whose production can be affected by inorganic ion receptor activity, preferably calcium receptor activity; (3) those characterized by an abnormal effect (*e.g.*, a different effect in kind or magnitude) of an intracellular or extracellular messenger which can itself be ameliorated by inorganic ion receptor activity, preferably calcium receptor activity; and (4) other diseases or disorders in which modulation of inorganic ion receptor activity, preferably calcium receptor activity, will exert a beneficial effect, for example, in diseases or disorders where the production of an intracellular or extracellular messenger stimulated by receptor activity compensates for an abnormal amount of a different messenger. Examples of extracellular messengers whose secretion and/or effect can be affected by modulating inorganic ion receptor activity include inorganic ions, hormones, neurotransmitters, growth factors, and chemokines. Examples of intracellular messengers include cAMP, cGMP, IP₃, and diacylglycerol.

In a preferred embodiment, the compound is used to treat a disease or disorder characterized by abnormal bone and mineral homeostasis, more preferably calcium homeostasis. Extracellular Ca²⁺ is under tight homeostatic control and controls various processes such as blood clotting, nerve and muscle excitability, and proper bone formation. Abnormal calcium homeostasis is characterized by one or more of the following activities: (1) an abnormal increase or decrease in serum calcium; (2) an abnormal increase or decrease in urinary excretion of calcium; (3) an abnormal increase or decrease in bone calcium levels, for example, as assessed by bone mineral density measurements; (4) an abnormal absorption of dietary calcium; (5) an abnormal increase or decrease in the production and/or release of messengers which affect serum calcium levels such as parathyroid hormone and calcitonin; and (6) an abnormal change in the response elicited by messengers which affect serum calcium levels.
The abnormal increase or decrease in these different aspects of calcium homeostasis is relative to that occurring in the general population and is generally associated with a disease or disorder.

Diseases and disorders characterized by abnormal calcium homeostasis can be due to different cellular defects such as a defective calcium receptor activity, a defective number of calcium receptors, or a defective intracellular protein acted on by a calcium receptor. For example, in parathyroid cells, the calcium receptor is coupled to the Gᵢ protein which in turn inhibits cyclic AMP production. Defects in Gᵢ protein can affect its ability to inhibit cyclic AMP production.

Diseases or disorders which can be treated by modulating calcium receptor activity are known in the art. For example, diseases or disorders which can be treated by modulating calcium receptor activity can be identified based on the functional responses of cells regulated by calcium receptor activity.

Functional responses of cells regulated by calcium receptor are know in the art, including PTH secretion by parathyroid cells, calcitonin secretion by C-cells, and bone resorption by osteoclasts. Such functional responses are associated with different diseases or disorders. For example, hyperparathyroidism results in elevated levels of PTH in the plasma. Decreasing the plasma levels of PTH offers an effective means of treating hyperparathyroidism. Likewise, increasing plasma levels of calcitonin is associated with an inhibition of bone resorption. Inhibiting bone resorption is an effective treatment for osteoporosis. Thus, modulation of calcium receptor activity can be used to treat diseases such as hyperparathyroidism, and osteoporosis.

Those compounds modulating inorganic ion receptor activity, preferably calcium receptor activity, can be used to confer beneficial effects to patients suffering from a variety of diseases or disorders. For example, osteoporosis is an age-related disorder characterized by loss of bone mass and increased risk of bone fracture. Compounds can be used to block osteoclastic bone resorption either directly (*e.g.*, an osteoclast ionomimetic compound) or indirectly by increasing endogenous calcitonin levels (*e.g.*, a C-cell calcimimetic). Alternatively, a calcilytic active on the parathyroid cell calcium receptor will increase circulating levels of parathyroid hormone, stimulating bone formation. All three of these approaches will result in beneficial effects to patients suffering from osteoporosis.

In addition, it is known that intermittent low dosing with PTH results in an anabolic effect on bone mass and appropriate bone remodeling. Thus, compounds and dosing regimens evoking transient increases in parathyroid hormone (*e.g.*, intermittent dosing with a parathyroid cell ionolytic) can increase bone mass in patients suffering from osteoporosis.

Additional diseases or disorders can be identified by identifying additional cellular functional responses, associated with a disease or disorder, which are regulated by calcium receptor activity. Diseases or disorder which can be treated by modulating other inorganic ion receptors can be identified in an analogous manner.

Different diseases can be treated by the present invention by targeting cells having a calcium receptor. For example, primary hyperparathyroidism (HPT) is characterized by hypercalcemia and abnormal elevated levels of circulating PTH. A defect associated with the major type of HPT is a diminished sensitivity of parathyroid cells to negative feedback regulation by extracellular Ca²⁺. Thus, in tissue from patients with primary HPT, the "set-point" for extracellular Ca²⁺ is shifted to the right so that higher than normal concentrations of extracellular Ca²⁺ are required to depress PTH secretion. Moreover, in primary HPT, even high concentrations of extracellular Ca²⁺ often depress PTH secretion only partially. In secondary (uremic) HPT, a similar increase in the set-point for extracellular Ca²⁺ is observed even though the degree to which Ca²⁺ suppresses PTH secretion is normal. The changes in PTH secretion are paralleled by changes in [Ca²⁺]ᵢ: the set-point for extracellular Ca²⁺-induced increases in [Ca²⁺]ᵢ is shifted to the right and the magnitude of such increases is reduced.

Patients suffering from secondary HPT may also have renal osteodystrophy. Calcimimetics appear to be useful for treating both abnormal PTH secretion and renal osteodystrophy in such patients.

Compounds that mimic the action of extracellular Ca²⁺ are beneficial in the long-term management of both primary and secondary HPT. Such compounds provide the added impetus required to suppress PTH secretion which the hypercalcemic condition alone cannot achieve and, thereby, help to relieve the hypercalcemic condition. Compounds with greater efficacy than extracellular Ca²⁺ may overcome the apparent nonsuppressible component of PTH secretion which is particularly troublesome in the major form of primary HPT caused by adenoma of the parathyroid gland. Alternatively, or additionally, such compounds can depress synthesis of PTH, as prolonged hypercalcemia has been shown to depress the levels of preproPTH mRNA in bovine and human adenomatous parathyroid tissue. Prolonged hypercalcemia also depresses parathyroid cell proliferation *in vitro,* so calcimimetics can also be effective in limiting the parathyroid cell hyperplasia characteristic of secondary HPT.

Cells other than parathyroid cells can respond directly to physiological changes in the concentration of extracellular Ca²⁺. For example, calcitonin secretion from parafollicular cells in the thyroid (C-cells) is regulated by changes in the concentration of extracellular Ca²⁺.

Isolated osteoclasts respond to increases in the concentration of extracellular Ca²⁺ with corresponding increases in [Ca²⁺]ᵢ that arise partly from the mobilization of intracellular Ca²⁺. Increases in [Ca²⁺]ᵢ in osteoclasts are associated with the inhibition of bone resorption. Release of alkaline phosphatase from bone-forming osteoblasts is directly stimulated by calcium.

Renin secretion from juxtaglomerular cells in the kidney, like PTH secretion, is depressed by increased concentrations of extracellular Ca²⁺. Extracellular Ca²⁺ causes the mobilization of intracellular Ca²⁺ in these cells. Other kidney cells respond to calcium as follows: elevated Ca²⁺ inhibits formation of 1,25(OH)₂-vitamin D by proximal tubule cells, stimulates production of calcium-binding protein in distal tubule cells, and inhibits tubular reabsorption of Ca²⁺ and Mg²⁺ and the action of vasopressin on the thick ascending limb of Henle's loop (MTAL), reduces vasopressin action in the cortical collecting duct cells, and affects vascular smooth muscle cells in blood vessels of the renal glomerulus.

Calcium also promotes the differentiation of intestinal goblet cells, mammary cells, and skin cells; inhibits atrial natriuretic peptide secretion from cardiac atria; reduces cAMP accumulation in platelets; alters gastrin and glucagon secretion; acts on vascular smooth muscle cells to modify cell secretion of vasoactive factors; and affects cells of the central nervous system and peripheral nervous system.

Thus, there are sufficient indications to suggest that Ca²⁺, in addition to its ubiquitous role as an intracellular signal, also functions as an extracellular signal to regulate the responses of certain specialized cells. Compounds of this invention can be used in the treatment of diseases or disorders associated with disrupted Ca²⁺ responses in these cells.

Specific diseases and disorders which might be treated or prevented, based upon the affected cells, also include those of the central nervous system such as seizures, stroke, head trauma, spinal cord injury, hypoxia-induced nerve cell damage such as in cardiac arrest or neonatal distress, epilepsy, neurodegenerative diseases such as Alzheimer's disease, Huntington's disease and Parkinson's disease, dementia, muscle tension, depression, anxiety, panic disorder, obsessive-compulsive disorder, post-traumatic stress disorder, schizophrenia, neuroleptic malignant syndrome, and Tourette's syndrome; diseases involving excess water reabsorption by the kidney such as syndrome of inappropriate ADH secretion (SIADH), cirrhosis, congestive heart failure, and nephrosis; hypertension; preventing and/or decreasing renal toxicity from cationic antibiotics (*e.g.,* aminoglycoside antibiotics); gut motility disorders such as diarrhea, and spastic colon; GI ulcer diseases; GI diseases with excessive calcium absorption such as sarcoidosis; and autoimmune diseases and organ transplant rejection.

While calcium receptor-modulating compounds of the present invention will typically be used in therapy for human patients, they may also be used to treat similar or identical diseases in other warm-blooded animal species such as other primates, farm animals such as swine, cattle, and poultry; and sports animals and pets such as horses, dogs and cats.

### IV. ADMINISTRATION

The compounds described by the present invention can be formulated for a variety of modes of administration, including systemic and topical or localized administration. Techniques and formulations generally may be found in Remington's Pharmaceutical Sciences, 18th ed., Mack Publishing Co., Easton, PA, 1990.

Suitable dosage forms, in part, depend upon the use or the route of entry, for example, oral, transdermal, transmucosal, or by injection (parenteral). Such dosage forms should allow the compound to reach a target cell whether the target cell is present in a multicellular host or in culture. For example, pharmacological compounds or compositions injected into the blood stream should be soluble. Other factors are known in the art, and include considerations such as toxicity and dosage forms which retard the compound or composition from exerting its effect.

Compounds can also be formulated as pharmaceutically acceptable salts and complexes thereof. Pharmaceutically acceptable salts are non-toxic salts in the amounts and concentrations at which they are administered. The preparation of such salts can facilitate the pharmacological use by altering the physical characteristics of the compound without preventing it from exerting its physiological effect. Useful alterations in physical properties include lowering the melting point to facilitate transmucosal administration and increasing the solubility to facilitate administering higher concentrations of the drug.

The pharmaceutically acceptable salt of the different compounds may be present as a complex. Examples of complexes include an 8-chlorotheophylline complex (analogous to, *e.g.*, dimenhydrinate:diphenhydramine 8-chlorotheophylline (1:1) complex; Dramamine) and various cyclodextrin inclusion complexes.

Pharmaceutically acceptable salts include acid addition salts such as those containing sulfate, hydrochloride, fumarate, maleate, phosphate, sulfamate, acetate, citrate, lactate, tartrate, methanesulfonate, ethanesulfonate, benzenesulfonate, p-toluenesulfonate, cyclohexylsulfamate and quinate. Pharmaceutically acceptable salts can be obtained from acids such as hydrochloric acid, maleic acid, sulfuric acid, phosphoric acid, sulfamic acid, acetic acid, citric acid, lactic acid, tartaric acid, malonic acid, methanesulfonic acid, ethanesulfonic acid, benzenesulfonic acid, p-toluenesulfonic acid, cyclohexylsulfamic acid, fumaric acid, and quinic acid.

Pharmaceutically acceptable salts also include basic addition salts such as those containing benzathine, chloroprocaine, choline, diethanolamine, ethylenediamine, meglumine, procaine, aluminum, calcium, lithium, magnesium, potassium, sodium, ammonium, alkylamine, and zinc, when acidic functional groups, such as carboxylic acid or phenol, are present. For example, see Remington's Pharmaceutical Sciences, 18th ed., Mack Publishing Co., Easton, PA, p. 1445, 1990. Such salts can be prepared using the appropriate corresponding bases.

Pharmaceutically acceptable salts can be prepared by standard techniques. For example, the free-base form of a compound is dissolved in a suitable solvent, such as an aqueous or aqueous-alcohol in solution containing the appropriate acid and then isolated by evaporating the solution. In another example, a salt is prepared by reacting the free base and acid in an organic solvent. (See, *e.g.*, PCT/US92/03736, hereby incorporated by reference herein.)

Carriers or excipients can also be used to facilitate administration of the compound. Examples of carriers include calcium carbonate, calcium phosphate, various sugars such as lactose, glucose, or sucrose, or types of starch, cellulose derivatives, gelatin, vegetable oils, polyethylene glycols and physiologically compatible solvents. Examples of physiologically compatible solvents include sterile solutions of water for injection (WFI), saline solution and dextrose.

The compounds can be administered by different routes including intravenous, intraperitoneal, subcutaneous, intramuscular, oral, topical (transdermal), or transmucosal administration. For systemic administration, oral administration is preferred. For oral administration, for example, the compounds can be formulated into conventional oral dosage forms such as capsules, tablets, and liquid preparations such as syrups, elixirs, and concentrated drops.

Alternatively, injection (parenteral administration) may be used, for example, intramuscular, intravenous, intraperitoneal, and/or subcutaneous administration. For injection, the compounds of the invention are formulated in liquid solutions, preferably, in physiologically compatible buffers or solutions, such as saline solution, Hank's solution, or Ringer's solution. In addition, the compounds may be formulated in solid form and redissolved or suspended immediately prior to use. Lyophilized forms can also be produced.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, bile salts and fusidic acid derivatives. In addition, detergents may be used to facilitate permeation. Transmucosal administration, for example, may be through nasal sprays, buccal or sublingual tablets, rectal suppositories, or vaginal suppositories.

For topical administration, the compounds of the invention can be formulated into ointments, salves, gels, or creams, as is generally known in the art.

The amounts of various compounds to be administered can be determined by standard procedures taking into account factors such as the compound IC₅₀, EC₅₀, the biological half-life of the compound, the age, size and weight of the patient, and the disease or disorder associated with the patient. The importance of these and other factors to be considered are known to those of ordinary skill in the art. Generally, it is an amount between about 0.01 and 50 mg/kg, preferably 0.01 and 20 mg/kg of the animal to be treated.

### V. EXAMPLES

### Example 1. Assaying Calcium Receptor Activity

The ability of different compounds to modulate calcium receptor activity are described in this example. Other methods which can be used to measure calcium receptor activity are known in the art.

Recombinant HEK 293 4.0-7 cells containing a calcium receptor were constructed as described by Rogers et al., J. Bone Miner. Res. 10 Suppl. 1:S483, 1995. The recombinant cells were loaded with fura-2 by incubating the cells in Dulbecco's modified Eagle's medium buffered with 20 mM HEPES containing about 5 µM fluo-3/AM for one hour at room temperature. Cells were then rinsed with Hank's balanced salt solution buffered with 20 mM HEPES containing 1 mM CaCl₂ and 1 mM MgCl₂. Compounds to be tested were then added to the cells and fluorescence was measured (excitation and emission wavelengths of 340 and 510 nm, respectively). Table I provides results for different compounds.

**Table I**

| Compound | EC₅₀(nM) |
|---|---|
| 27H | 2750 |
| 28G | 602 |
| 28H | 3000 |
| 28I | 1200 |
| 28J | 1100 |
| 28K | 57 |

### Reference Example 2: Synthesis of 26D. (R,R)-N-(1-Ethyl-4'-iodophenyl)-1-(1-naphthyl)ethylamine hydrochloride

The synthesis of the title compound (26D) was accomplished in a one-pot, two-step reaction sequence by reductive amination of the imine formed from the commercially available 4'-iodoacetophenone and (*R*)-naphthyl-1-ethylamine. The reduction of the imine diastereoselectively was conducted under similar conditions as previously reported (Tetrahedron Lett. (1985) 41, 6005-6011.).

A mixture of 4'-iodoacetophenone (0.25 g, 1.0 mmol), (*R*)-naphthyl-1-ethylamine (0.17 g, 1.0 mmol), and Ti(i-PrO), (0.38 mL, 1.1 mmol) in abs. EtOH (5 mL) was refluxed for 18 h. Diethyl-1,4-dihydro-2,6-dimethyl-3,5-pyridine decarboxylate (0.25 g, 1.0 mmol) and Mg(ClO₄)₂ (0.22 g, 1.0 mmol) were then added to the reaction mixture and the reflux was continued for an additional 18 h. The reaction mixture was then cooled to ambient temperature, H₂O (3 mL) and diethyl ether (10 mL) were added and the mixture was centrifuged (3000 rpm) to remove the inorganic salts. The supernatant was decanted away from the pellet and the volatiles were removed under reduced pressure. The resulting residue was chromatographed on silica gel (elution with 1% MeOH/CH₂Cl₂) to provide the purified product as its free base. This material was converted to its hydrochloride salt. The salt was recrystallized from CH₂Cl₂/hexane to provide GC/MS-pure material.

### Reference Example 3: Synthesis of 26E (R,R)-N-(1-Ethyl-4'-ethoxy-3'-methylphenyl)-1-(1-naphthyl)ethylamine hydrochloride

The synthesis of the title compound (26E) was accomplished in a three-step, two-pot reaction sequence. Commercially available 4-hydroxy-3-methylacetophenone was *O*-alkylated with ethyl iodide/K₂CO₃/acetone. This ketone was subsequently reacted with (*R*)-naphthyl-1-ethylamine in the presence of Ti(i-PrO)₄ to provide the imine. This imine was reduced in high diastereoselective yield by catalytic hydrogenation with Raney-nickel.

A mixture of 4-ethoxy-3-methylacetophenone (2.0 g, 11.2 mmol), (*R*)-naphthyl-1-ethylamine (2.0 g, 11.2 mmol), Ti(i-Pro)₄ (4.2 mL, 14.1 mmol), and EtOH (10 mL) were stirred at 60 °C for 18 h. The reaction mixture was then transferred to a Parr hydrogenation flask, Raney-nickel (100 mg; washed with EtOH, 3 x 20 mL) was added, and the mixture was hydrogenated at 50 psig, 25 °C, for 4 h. The reaction mixture was then filtered (Celite/fritted glass), the catalyst was washed (EtOH, 20 mL), and the filtrate was evaporated under reduced pressure to provide the crude product. This material was purified by silica gel chromatography (elution with 2% MeOH/CH₂Cl₂). The free base was converted to its hydrochloride salt to provide 1.1 g (27%) of a white solid.

### Reference Example 4: Synthesis of 26F, (R,R)-N-(1-Propyl-4'-methoxy-3'-methylphenyl)-1-(1-naphthyl)ethylamine hydrochloride

The synthesis of the title compound (26F) was accomplished in a four-step, three-pot reaction sequence. Commercially available 3-methyl-p-anisaldehyde was reacted with ethylmagnesium bromide to provide its phenylpropanol derivative. This alcohol was then oxidized to the corresponding ketone in the usual manner with PCC. This ketone was subsequently reacted with (*R*)-naphthyl-1-ethylamine in the presence of Ti(i-PrO)₄ to provide the imine. This imine was reduced in high diastereoselective yield by catalytic hydrogenation in the presence of Raney-nickel.

In a manner similar to the synthesis of 26E, a mixture of 4-methoxy-3-methylpropiophenone (5.7 g, 31.7 mmol), (*R*)-naphthyl-1-ethylamine (5.2 mL, 31.7 mmol), Ti(i-Pro)₄ (11.8 mL, 39.6 mmol), and EtOH (30 mL) were reacted as above to form the imine which was subsequently reduced under catalytic hydrogenation conditions over Raney-nickel. The crude product was purified by silica gel chromatography (elution with 10:1, hexane/EtOAc). The free base was converted to its hydrochloride salt to provide 0.50 g (4%) of a white solid.

### Reference Example 5: Synthesis of 15G, (R,R)-N-(1-Ethyl-4'-methoxy-3'-bromophenyl)-1-(1-naphthy-1-ethylamine hydrochloride

The synthesis of the title compound (26G) was accomplished in a four-step, three-pot reaction sequence. Commercially available 3-bromo-4-methoxybenzaldehyde was reacted with methylmagnesium bromide to provide its phenylethanol derivative. This alcohol was then oxidized to the corresponding ketone in the usual manner with pyridinium chlorochromate (PCC). This ketone was subsequently reacted with (*R*)-naphthyl-1-ethylamine in the presence of Ti(i-PrO)₄ to provide the imine. This imine was reduced in high diastereoselective yield using diethyl-1,4-dihydro-2,6-dimethyl-3,5-pyridine decarboxylate.

In a manner similar to the synthesis of 26D, a mixture of 3-bromo-4-methoxyacetophenone (3.0 g, 13.1 mmol), (*R*)-naphthyl-1-ethylamine (2.1 mL, 13.1 mmol), and Ti(i-PrO)₄ (4.7 mL, 15.7 mmol) in abs. EtOH (100 mL) was reduced with diethyl-1,4-dihydro-2,6-dimethyl-3,5-pyridine decarboxylate in the presence of Mg(ClO₄)₂. The resulting crude material was converted to its hydrochloride salt. The salt was purified by precipitation from diethyl ether/hexane to provide GC/MS-pure material (0.6 g, 11%) as a white solid.

### Reference Example 6: Synthesis of 26H, 26I, and 26J. (R)-N-(3-phenyl-2-propenyl)-1-(1-naphthyl)ethylamine hydrochloride, (R)-N-(2-methyl-3-phenyl-2-propenyl)-1-(1-naphthyl)ethylamine hydrochloride, and (R)-N-(2-methoxy-3-phenyl-2-propenyl)-1-(1-naphthyl)ethylamine hydrochloride

The syntheses of the title compounds were accomplished in three, two-step, one-pot reaction sequences. Commercially available cinnamaldehyde, 2-methyl-trans-cinnamaldehyde, and 2-methoxycinnamaldehyde, respectively, were reacted with (*R*)-naphthyl-1-ethylamine in the presence of Ti(i-PrO)₄ to provide the corresponding imine. These imines were reduced using sodium cyanoborohydride to provide the title compounds in high overall yields.

### Example 7: Physical Data

Table II provides physical data for some of the compounds described herein. Gas chromatographic and mass spectral data were obtained on a Hewlett-Packard 5890 Series II Gas Chromatograph with a 5971 Series Mass Selective Detector [Ultra-2 Ultra Performance Capillary Column (crosslinked 5% Ph Me silicone); column length, 25 m, column i.d., 0.20 mm, film thickness, 0.33 µm; He flow rate, 60 mL/min; injector temp., 250 °C; temp. program, 20 °C/min from 125 to 325 °C for 10 min, then held constant at 325 °C for 6 min].

**TABLE II**

| Coumpound | GCᵣₜ | *m*/*z* |
|---|---|---|
| 26G | 9.09 | 385 |
| 26H | 10.95 | 287 |
| 26I | 10.98 | 301 |
| 26J | 11.79 | 317 |

Additional Gas chromatographic and mass spectral data were obtained on a Hewlett-Packard 5890 Series II Gas Chromatograph with a 5971 Series Mass Selective Detector [Ultra-2 Ultra Performance Capillary Column (crosslinked 5% phenyl methyl silicone); column length, 25 m, column i.d., 0.20 mm; He flow rate, 60 mL/min; injector temp., 250 °C; gradient temperature program, 20 °C/min from 125 to 325 °C for 10 min, then held constant at 325 °C for 6 min].

Compound 28G, rt = 7.18', m/z (rel. int.) 251 (M+,6), 236 (43), 156 (6), 155 (26), 154 (32), 153 (24), 152 (18), 152 (18). 151 (6), 141 (8), 129 (11), 128 (25), 127 (31), 126 (11), 115 (12), 95 (12), 82 (6), 81 (100), 77 (8), 53 (27), 51 (6).

Compound 28H, rt = 7.31', m/z (rel. int.) 251 (M+,9), 236 (200), 208 (7), 170 (10), 168 (8), 156 (5), 155 (26), 154 (39), 153 (27), 152 (19), 152 (19), 151 (6), 141 (8), 129 (9), 128 (22), 127 (29), 126 (10), 115 (9), 94 (5), 82 (5), 81 (77), 53 (13).

Compound 28I, rt = 8.20', m/z (rel. int.) 267 (M+,6), 252 (36), 156 (6), 155 (21), 154 (15), 153 (15), 152 (10), 141 (7), 129 (7), 128 (15), 127 (16), 126 (5), 115 (8), 112 (16), 98 (8), 98 (8), 98 (6), 96 (100), 53 (5), 44 (6).

Compound 28J, rt = 8.23', m/z (rel. int.) 267 (M+,6), 251 (56), 170 (11), 155 (25), 154 (31), 153 (23), 153 (23), 152 (16), 151 (5), 141 (7), 129 (9), 128 (22), 127 (26), 126 (9), 115 (10), 111 (7), 110 (7), 98 (6), 97 (8), 96 (100), 85 (5), 77 (5), 53 (6), 44 (9).

## Claims

1. An inorganic ion receptor-modulating compound having the formula: 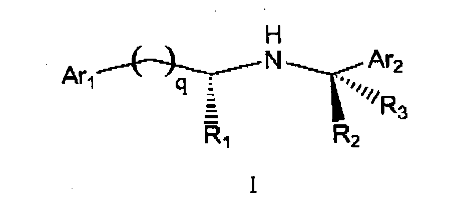
wherein Ar₁ is an optionally substituted naphthyl or heterocyclic aryl, wherein up to 5 substituents may be present and each substituent is independently selected from alkyl, alkenyl, halogen, alkoxy, thioalkyl, methylene dioxy, haloalkyl, haloalkoxy, OH, CH₂OH, CONH₂, CN, acetoxy, N(alkyl)₂, phenyl, phenoxy, benzyl, benzyloxy, α,α-dimethylbenzyl, NO₂, CHO, CH₃CH(OH), acetyl, OCH₂COOH, and ethylene dioxy;
Ar₂ is an optionally substituted naphthyl or heterocyclic aryl, wherein up to 5 substituents may be present and each substituent is independently selected alkyl, alkenyl, halogen, alkoxy, thioalkyl, methylene dioxy, haloalkyl, haloalkoxy, OH, CH₂OH, CONH₂, CN, OCH₂COOH, ethylene dioxy, and acetoxy;
wherein one or both of Ar₁ and Ar₂ are heterocyclic aryl;
q is 0, 1, 2, or 3;
R₁ is either H or alkyl;
R₂ and R₃ are each independently either hydrogen, alkyl, or together cycloalkyl or cycloalkenyl;
and pharmaceutically acceptable salts and complexes thereof;
wherein said compound is an ionomimetic modulating one or more inorganic ion receptor activities.

2. The compound of claim 1, wherein Ar₁ is an optionally substituted heterocyclic aryl selected from furanyl, thiofuranyl, benzofuranyl, and benzothiophenyl.

3. The compound of claim 2, wherein R₂ and R₃ are each independently hydrogen, alkyl, or together cycloalkyl, provided that at least one of R₂ and R₃ is not hydrogen.

4. The compound of claim 3, wherein R₂ is not hydrogen.

5. The compound of claim 4, wherein R₂ and R₃ are both methyl.

6. The compound of any of claims 1-4, wherein R₃ is hydrogen.

7. The compound of any of claims 1-6, wherein R₁ is alkyl.

8. The compound of claim 7, wherein said R₁ alkyl has more than one carbon atoms.

9. The compound of any of claims 1-6, wherein R₁ is hydrogen.

10. The compound of any of claims 1-9, wherein Ar₂ is an optionally substituted naphthyl.

11. The compound of claim 10, wherein Ar₂ is an unsubstituted naphthyl.

12. The compound of claim 10, wherein Ar₂ is a substituted naphthyl.

13. The compound of claim 12, wherein Ar₂ is a substituted naphthyl having 1 to 4 independently selected substituents.

14. The compound of claim 12, wherein Ar₂ is a substituted naphthyl having 1 to 4 independently selected substituents selected from isopropyl, CH₃O, CH₃S, CF₃O, Br, I, Cl, F, CF₃, and CH₃.

15. The compound of claim 12, wherein Ar₂ is a substituted naphthyl having 1 substituent selected from isopropyl, CH₃O, CH₃S, CF₃O, Br, I, Cl, F, CF₃, and CH₃

16. The compound of any of claims 1-15, wherein q is 2.

17. The compound of any of claims 1-15, wherein q is 0.

18. A pharmaceutical composition comprising the compound of any of claims 1-17 and a pharmaceutical acceptable carrier.

19. A compound of any of claims 1-18 for use as a medicament.

20. A compound of any of claims 1-18 for treatment of a disease **characterized by** either, or both, of (1) abnormal calcium homeostasis, and (2) an abnormal amount of an extracellular or intracellular messenger whose production can be affected by calcium receptor activity..

21. The compound of claim 20, wherein said disease is selected from a group consisting of primary and secondary hyperparathyroidism, Paget's disease, hypercalcemia malignancy, osteoporosis, hypertension, and renal osteodystrophy.

22. The compound of claim 20, wherein said disease is selected from the group consisting of primary and secondary hyperparathyroidism.

23. The compound of any of claims 1-18 for decreasing serum PTH in a patient.

24. The compound of claim 23, wherein serum PTH level is reduced to a degree sufficient to cause a decrease in plasma Ca²⁺.

## Patentansprüche

1. Eine anorganische Ionenrezeptor modulierende Verbindung mit der Formel: 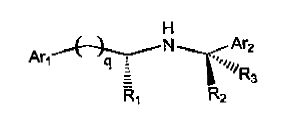
in der Ar₁ ein optional substituiertes Naphtyl oder heterozyklisches Aryl ist, in dem bis zu fünf Substituenten vorhanden sein können und jeder Substituent ist unabhängig aus Alkyl, Alkenyl, Halogen, Alkyloxy, Thioalkyl, Methylendioxy, Haloalkyl, Haloalkyloxy, OH, CH₂OH, CONH₂, CN, Acetoxy, N(Alkyl)₂, Phenyl, Phenoxy, Benzyl, Benzyloxy, α,α-Dimethylbenzyl, NO₂, CHO, CH₃CH(OH), Acetyl, OCH₂COOH und Ethylendioxy ausgewählt,
in der Ar₂ ein optional substituiertes Naphthyl oder heterozyklisches Aryl ist, in dem bis zu fünf Substituenten vorhanden sein können und jeder Substituent ist unabhängig aus Alkyl, Alkenyl, Halogen, Alkyloxy, Thioalkyl, Methylendioxy, Haloalkyl, Haloalkyloxy, OH, CH₂OH, CONH₂, CN, OCH₂COOH, Ethylendioxy und Acetoxy ausgewählt,
in der einer oder beide von Ar₁ und Ar₂ ein heterozyklisches Aryl sind, während q 0,1, 2 oder 3 ist,
R₁ entweder H oder ein Alkyl ist,
R₂ und R₃ jeweils unabhängig entweder Wasserstoff oder ein Alkyl oder zusammen ein Cykloalkyl oder Cykloalkenyl sind,
sowie pharmazeutisch geeignete Salze und Komplexe davon,
wobei die Verbindung eine ionenmimetisch Modulierende einer oder mehrere anorganischer Rezeptoraktivitäten ist.

2. Die Verbindung gemäß Anspruch 1, in der Ar₁ ein optional substituiertes heterozyklisches Aryl ausgewählt aus Furanyl, Thiofuranyl, Benzofuranyl und Benzothiophenyl ist.

3. Die Verbindung gemäß Anspruch 2, in der R₂ und R₃ jeweils unabhängig Wasserstoff oder ein Alkyl oder zusammen Cykloalkyl sind, wenn sichergestellt ist, dass mindestens einer von R₂ und R₃ nicht Wasserstoff ist.

4. Die Verbindung gemäß Anspruch 3, in der R₂ nicht Wasserstoff ist.

5. Die Verbindung gemäß Anspruch 4, in der R₂ und R₃ beide Methyl sind.

6. Die Verbindung gemäß einem der Ansprüche 1 bis 4, in der R₃ Wasserstoff ist.

7. Die Verbindung gemäß einem der Ansprüche 1 bis 6, in der R₁ ein Alkyl ist.

8. Die Verbindung gemäß Anspruch 7, in der das R₁-Alkyl mehr als ein Kohlenstoffatom aufweist.

9. Die Verbindung gemäß einem der Ansprüche 1 bis 6, in der R₁ Wasserstoff ist.

10. Die Verbindung gemäß einem der Ansprüche 1 bis 9, in der Ar₂ ein optional substituiertes Naphtyl ist.

11. Die Verbindung gemäß Anspruch 10, in der Ar₂ ein nicht substituiertes Naphtyl ist.

12. Die Verbindung gemäß Anspruch 10, in der Ar₂ ein subtituiertes Naphtyl ist.

13. Die Verbindung gemäß Anspruch 12, in der Ar₂ ein substituiertes Naphtyl mit eins bis vier unabhängig ausgewählten Substituenten ist.

14. Die Verbindung gemäß Anspruch 12, in der Ar₂ ein substituiertes Naphtyl mit eins bis vier unabhängig ausgewählten Substituenten ausgewählt aus Isopropyl, CH₃O, CH₃S, CF₃O, Br, I, CI, F, CF₃ und CH₃ ist.

15. Die Verbindung gemäß Anspruch 12, in der Ar₂, ein substiuiertes Naphtyl mit einem Substituenten ausgewählt aus Isopropyl, CH₃O, CH₃S, CF₃O, Br, I, CI, F, CF₃ und CH₃ ist.

16. Die Verbindung gemäß einem der Ansprüche 1 bis 15, in der q 2 ist.

17. Die Verbindung gemäß einem der Ansprüche 1 bis 15, in der q 0 ist.

18. Eine pharmazeutische Zusammensetzung, die die Verbindung gemäß einem der Ansprüche 1 bis 17 und einen pharmazeutisch akzeptablen Träger umfasst.

19. Eine Verbindung gemäß einem der Ansprüche 1 bis 18 für die Verwendung als ein Medikament.

20. Eine Verbindung gemäß einem der Ansprüche 1 bis 18 zur Behandlung einer Krankheit, die charakterisiert ist durch: (1) eine abnorme Kalziumhomöostase und/oder (2) eine abnorme Menge an extrazellulären oder intrazellulären Boten, deren Produktion durch eine Kalziumrezeptoraktivität beeinflusst werden kann.

21. Die Verbindung gemäß Anspruch 20, in der die Krankheit aus der Gruppe bestehend aus primärem und sekundärem Hyperparathyreoidismus, Morbus Paget, malignischer Hypercalcämie, Osteoporose, Hypertonie und Nierenosteodystrophie ausgewählt ist.

22. Die Verbindung gemäß Anspruch 20, in der die Krankheit aus der Gruppe bestehend aus primärem und sekundärem Hyperparathyreoidismus ausgewählt ist.

23. Die Verbindung gemäß einem der Ansprüche 1 bis 18, um Serum-PTH in einem Patienten abzusenken.

24. Die Verbindung gemäß Anspruch 23, in der der Serum-PTH-Spiegel auf einen Grad reduziert ist der genügt, um eine Absenkung im Plasma Ca²⁺ zu verursachen.

## Revendications

1. Composé modulateur de récepteur d'ion inorganique ayant la formule : 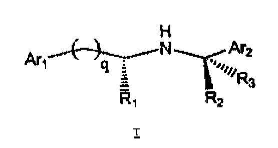
où Ar₁ est un naphtyle ou un aryle hétérocyclique facultativement substitué, où jusqu'à 5 substituant peuvent être présents et chaque substituant est indépendamment choisi parmi un alkyle, un alcényle, un halogène, un alcoxy, un thioalkyle, un méthylènedioxy, un halogénoalkyle, un halogénoalcoxy, OH, CH₂OH, CONH₂, CN, un acétoxy, N(alkyle)₂, un phényle, un phénoxy, un benzyle, un benzyloxy, un α,α-diméthylbenzyle, NO₂, CHO, CH₃CH(OH), un acétyle, OCH₂COOH, et un éthylènedioxy ;
Ar₂ est un naphtyle ou un aryle hétérocyclique facultativement substitué, où jusqu'à 5 substituants peuvent être présents et chaque substituant est indépendamment choisi parmi un alkyle, un alcényle, un halogène, un alcoxy, un thioalkyle, un méthylènedioxy, un halogénoalkyle, un halogénoalcoxy, OH, CH₂OH, CONH₂, CN, OCH₂COOH, un éthylènedioxy et un acétoxy ;
où l'un ou les deux parmi Ar₁ et Ar₂ sont un aryle hétérocyclique ;
q est 0, 1, 2 ou 3 ;
R₁ est H ou un alkyle ;
R₂ et R₃ sont chacun indépendamment un hydrogène, un alkyle, ou conjointement un cycloalkyle ou un cycloalcényle ;
et des sels et complexes pharmaceutiquement acceptables de celui-ci;
**caractérisé en ce que** ledit composé est un ionomimétique modulant une ou plusieurs activités de récepteur d'ion inorganique.

2. Composé de la revendication 1, **caractérisé en ce que** Ar₁ est un aryle hétérocyclique facultativement substitué choisi parmi le furanyle, le thiofuranyle, le benzofuranyle et le benzothiophényle.

3. Composé de la revendication 2, **caractérisé en ce que** R₂ et R₃ sont chacun indépendamment un hydrogène, un alkyle ou conjointement un cycloalkyle, à condition qu'au moins l'un de R₂ et R₃ ne soit pas un hydrogène.

4. Composé de la revendication 3, **caractérisé en ce que** R₂ n'est pas un hydrogène.

5. Composé de la revendication 4, **caractérisé en ce que** R₂ et R₃ sont tous deux un méthyle.

6. Composé de l'une quelconque des revendications 1 à 4, **caractérisé en ce que** R₃ est un hydrogène.

7. Composé de l'une quelconque des revendications 1 à 6, **caractérisé en ce que** R₁ est un alkyle.

8. Composé de la revendication 7, **caractérisé en ce que** ledit alkyle R₁ a plus d'un atome de carbone.

9. Composé de l'une quelconque des revendications 1 à 6, **caractérisé en ce que** R₁ est un hydrogène.

10. Composé de l'une quelconque des revendications 1 à 9, **caractérisé en ce que** Ar₂ est un naphtyle facultativement substitué.

11. Composé de la revendication 10, **caractérisé en ce que** Ar₂ est un naphtyle non substitué.

12. Composé de la revendication 10, **caractérisé en ce que** Ar₂ est un naphtyle substitué.

13. Composé de la revendication 12, **caractérisé en ce que** Ar₂ est un naphtyle substitué ayant de 1 à 4 substituants indépendamment choisis.

14. Composé de la revendication 12, **caractérisé en ce que** Ar₂ est un naphtyle substitué ayant 1 à 4 substituants indépendamment choisis parmi l'isopropyle, CH₃O, CH₃S, CF₃O, Br, I, Cl, F, CF₃ et CH₃.

15. Composé de la revendication 12, **caractérisé en ce que** Ar₂ est un naphtyle substitué ayant 1 substituant choisi parmi l'isopropyle, CH₃O, CH₃S, CF₃O, Br, I, Cl, F, CF₃ et CH₃.

16. Composé de l'une quelconque des revendications 1 à 15, **caractérisé en ce que** q est 2.

17. Composé de l'une quelconque des revendications 1 à 15, **caractérisé en ce que** q est 0.

18. Composition pharmaceutique comprenant le composé de l'une quelconque des revendications 1 à 17 et un véhicule pharmaceutiquement acceptable.

19. Composé de l'une quelconque des revendications 1 à 18 pour utilisation en tant que médicament.

20. Composé de l'une quelconque des revendications 1 à 18 pour le traitement d'une maladie **caractérisée par** l'un ou les deux parmi (1) une homéostasie du calcium anormale, et (2) une quantité anormale d'un messager extracellulaire ou intracellulaire dont la production peut être affectée par une activité de récepteur de calcium.

21. Composé de la revendication 20, **caractérisé en ce que** ladite maladie est choisie dans le groupe constitué de l'hyperparathyroïdisme primaire et secondaire, la maladie de Paget, l'hypercalcémie maligne, l'ostéoporose, l'hypertension et l'ostéodystrophie rénale.

22. Composé de la revendication 20, **caractérisé en ce que** ladite maladie est choisie dans le groupe constitué de l'hyperparathyroïdisme primaire et secondaire.

23. Composé de l'une quelconque des revendications 1 à 18 pour diminuer la parathormone sérique chez un patient.

24. Composé de la revendication 23, **caractérisé en ce que** le taux sérique de parathormone est réduit à un degré suffisant pour causer une diminution de Ca²⁺ plasmatique.
